Europäisches Patentamt

European Patent Office

Office européen des brevets

(11) Publication number: **0 322 082**
**A1**

## EUROPEAN PATENT APPLICATION

(21) Application number: **88203007.5**

(22) Date of filing: **23.12.88**

(51) Int. Cl.4: **C12P 21/02 , C12N 9/00 , C12N 9/28 , C12N 9/54**

(30) Priority: **23.12.87 EP 87202620**

(43) Date of publication of application:
**28.06.89 Bulletin 89/26**

(84) Designated Contracting States:
**AT BE CH DE ES FR GB GR IT LI LU NL SE**

(71) Applicant: **GIST-BROCADES N.V.**
**Wateringseweg 1**
**NL-2611 XT Delft(NL)**

(72) Inventor: **De Boer, Willem Roelf**
**J. Evertsenlaan 3**
**NL-2341 St. Oegstgeest(NL)**
Inventor: **Van Heil, Bart**
**2e Oosterparkstraat 47**
**NL-1091 HW Amsterdam(NL)**
Inventor: **Schipper, Dirk**
**Oostsingel 205**
**NL-2612 HL Delft(NL)**
Inventor: **Spinnewijn, Octaaf**
**Mijdoornstraat 21**
**NL-2636 BJ Schipluiden(NL)**
Inventor: **Zuidweg, Marinus Hendrik Johan**
**Holwardastraat 20**
**NL-3328 HC Dordrecht(NL)**

(74) Representative: **Huygens, Arthur Victor, Dr. et al**
**c/o Gist-Brocades N.V. Patent & Trademarks Department Wateringseweg 1 PO Box 1**
**NL-2600 MA Delft(NL)**

(54) **Purified industrial enzyme and a process for the preparation thereof.**

(57) An industrial extracellular enzyme from the fermentation of a Bacillus strain, substantially free from cell wall polymers, is provided. Cell wall polymers are removed by ion exchange chromatography. The purified enzymes have reduced allergenicity and cause less haze formation when formulated in detergent compositions.

## Purified industrial enzyme and process for the preparation thereof

This invention relates to a purified industrial enzyme from the fermentation of a Bacillus strain and to a process of preparing the same.

Bacteria of the genus Bacillus produce a wide variety of extracellular enzymes, for example amylases, proteases, gluconases and lipases (F.G. Priest, Bact. Rev. 41, 711-753 (1977)). These enzymes have many applications, for example they are used in detergents or in the production of glucosyrups applied in food and drinks. Nowadays Bacilli are also used as a host cell system for the production of foreign proteins (W.E. Workman, J.H. McLinden and D.H. Dean, Crit. Rev. Biotechn. 3, 199 (1986)). One of the major goals of using Bacillus for cloning is to take advantage of the protein secretory system to translocate the foreign proteins to the culture medium, thereby facilitating purification.

Gram-positive Bacilli have a cell wall of relatively simple structure. One continuous layer of 20-60 nm thickness encloses the cytoplasmic membrane (A.M. Glauert and M.J. Thornley, Ann. Rev. Microbiol. 23, 159-198 (1969)) approximately half of it being peptidoglycan. The other half of the wall is made up by one of a variety of negatively charged polymers (the so-called anionic polymers) which are covalently linked to the peptidoglycan.

Peptidoglycan is a polysaccharide substituted with oligopeptide side chains. The polymer backbone is formed by N-acetylglycosamine and its 3-0-D-lactyl ether derivative, N-acetylmuramic acid, occurring in an alternating sequence and connected by $\beta$-(1-4) linked glucosidic bonds. Most of the hydroxyl groups in the lactyl residue of N-acetylmuramic acid are substituted by short peptide chains containing sequentially L-alanine, D-isoglutamic acid, meso-diaminopimelic acid and D-alanine which latter component is linked to a great extent to the $\epsilon$-amine group of the diaminopimelic acid in a neighbouring chain (H.G. Schlegel, Algemeine Mikrobiologie, Auflage 5, (1981)).

Besides peptidoglycan, the cell wall also contains anionic polymers. They may comprise a substantial part of the cell wall (up to 60%), (D.C. Ellwood and P.W. Tempest, Adv. Microbiol. Physiol. 7, 83-117 (1972); F.J. Kruyssen, W.R. de Boer and J.T.M. Wouters, J. Bacteriol. 144, 238-246 (1980)) and can be divided into two classes based on the nature of their negatively charged groups. Most abundant are the teichoic acids which contain phosphate groups as a part of the backbone of the polymer. There is a great structural diversity within this group of cell wall polymers. Generally, all polymers possessing phosphodiester groups, polyols and/or sugar residues are now classified as teichoic acids (J. Baddiley, Essays Biochem. 7, 35-77 (1972)). The second group of anionic acids encompasses the teichuronic acids. The structure of these anionic polymers is less well known than that of the teichoic acids. In B. subtilis - (W.R. de Boer, Ph. D. Thesis, University of Amsterdam (1979); J. Wright and J.E. Heckels, Biochem. J. 147, 187-189 (1975)) and B. licheniformis (M.R. Lifely, E. Tarelli and J. Baddiley, Biochem. J. 191, 305-308 (1980)) the teichuronic acids are mostly composed of equimolar amounts of D-glucuronic acid and N-acetylgalactosamine.

One of the common metabolic features of Bacillus strains is the loss of cell wall polymers during growth, a phenomenon generally called cell wall turnover (W.R. de Boer, Ph.D. Thesis, University of Amsterdam (1979); W.R. de Boer, F.J. Kruyssen and J.T.M. Wouters, J. Bacteriol. 140, 50-60 (1981); P.D. Meyer, Ph.D. Thesis, University of Amsterdam (1985); A.L. Kock and R.J. Doyle, FEMS Microbiol. Rev. 32, 247-254 (1986)). Cell wall turnover is considered to be part of the normal physiology of the cells and has been shown to occur in many strains of Bacillus. One consequence of cell wall turnover is the release of peptidoglycan, teichoic acid and teichuronic acid into the medium. The molecular weights of these polymers may be variable, but complexes can be formed with molecular weights in the same range as the extracellular proteins. Therefore, purification methods for extracellular proteins produced in fermentation by Bacillus strains (e.g. ultrafiltration), which are often based on molecular weight separations, are likely to result in accumulation of cell wall polymers and the desired extracellular protein in one single fraction. Analysis of a number of enzyme preparations produced by Bacillus indeed showed the presence of all three polymers in very significant amounts, teichoic acid being the predominant component.

Surprisingly, it appeared that removing cell wall polymers in enzyme preparations a number of hard properties of these enzymes disappeared and that the presence of these cell wall polymers in enzyme preparations appeared to be undesirable for the following reasons:
- these components may contribute to the allergenicity of the product either by direct immunogenic activity or by acting as an adjuvant for other immunogenic components (K.W. Knox and A.J. Wicken, Bact. Rev. 37-(2), 215-257 (1973));
- due to their high concentration in final enzyme preparations, they may have adverse effects on the enzyme stability (e.g. by cation binding) and on ultrafiltration properties of the enzyme (possibly leading to lower final enzyme concentration);

- in those cases where enzyme/protein productivity of the strains is lower than in strains used at present for protease/amylase production (e.g. during production of heterologous enzymes by Bacillus), the ratio between enzyme and cell wall polymers will even be more unfavourable, and removal of the cell wall polymers will be a necessity during purification;

- a protein preparation containing teichoic acid, when added to detergent solutions, may cause haze formation.

The process of purifying proteins from cell wall polymers has to be applicable on an industrial scale. Industrial scale is defined throughout this patent specification as applying mash of more than 1000 1.

Therefore, an object of the present invention is to provide an industrial extracellular protein (e.g. enzyme) being substantially free of cell wall polymers and to provide a process to prepare such a product.

In accordance with the present invention it was now found that the usefullness of such proteins (e.g. enzymes) could be improved by removing cell wall polymers, for instance by ion exchange chromatography. In this way, industrial extracellular enzymes from the fermentation of a Bacillus strain substantially free from cell wall polymers could be obtained.

The invention is particularly applicable to the production of purified industrial $\alpha$-amylase, more particularly of an industrial thermostable amylase substantially free from cell wall polymers.

In a preferred embodiment, an industrial $\alpha$-amylase is provided containing less than 0.2 mMol teichoic acid per $10^6$ TAU, less than 0.1 mMol glucosamine per $10^6$ TAU, less than 0.03 mMol muramic acid per $10^6$ TAU, less than 0.2 mMol diaminopimelic acid per $10^6$ TAU, less than 0.1 mMol galactosamine per $10^6$ TAU and less than 0.1 mMol glucuronic acid per $10^6$ TAU. TAU (the unit of thermostable amylase activity) is defined below.

The invention is also particularly applicable to the production of purified industrial protease, more particularly of an industrial protease active in alkaline medium.

The invention will be further illustrated, for the purpose of example, by the following reference to removing the cell wall polymers to a large extent by ion exchange chromatography: a fermentation broth of a Bacillus licheniformis producing an enzyme is analysed for enzyme activity, protein content and cell wall polymer concentration. A final sample is treated, in an extra recovery step, by ion exchange chromatography. For instance, a column containing a strong macro-reticular acryl resin is used and as eluent a solution with a pH of 5-7. This method proved to be very effective in separating cell wall polymers from the amylase enzyme with about 90% of the teichoic acid bound to the ion exchange column, from which it can be eluted completely in a later step. Ion exchange treated and untreated samples are compared for allergenicity, enzyme properties and filtration properties of the preparation.

Before the extra recovery step, the pH of the sample is adjusted to a pH of about 5-7. The pH may be modified by any convenient means, such as the addition of acetic acid, or other convenient acid or buffers, usually at a normality in the range of about 0.1 to concentrated.

During the ion exchange step, the sample may be combined with the ion exchange resin either batchwise or continuously through a column, where contact will normally be maintained for from about 5-60 min, preferably 10-30 min. An anion exchange adsorbent is employed, such as QAE, bound to a commercially available carrier.

A wide variety of supports and adsorbents may be used as the solid carriers or supports. Such solid carriers include inorganic carriers, such as glass and silica gel, organic, synthetic or naturally-occurring carriers, such as agarose, cellulose, dextran, polyamide, polyacrylamides, vinyl copolymers of bifunctional acrylates, and various hydroxylated monomers, and the like, with a suitable pore size. Commercialy available carriers are sold under the names of Sephadex®, Trisacryl®, Ultrogel®, Dynospheres®, Macrosorb®, XAD resins, and others.

The amylase enzyme activity is expressed in TAU units. One unit of thermostable amylase activity (TAU) as used herein is the amount of enzyme which hydrolyses 1.0 mg of soluble starch (100% of dry matter) per minute under standard conditions (pH 6.6, 30° C) into a product which, after reaction with an iodine solution of known strength, gives an optical density at 620 nm equivalent to that of a colour reference as described in the Iodine Starch Amylase Test described below.

The protease enzyme activity is expressed in ADU (Alkaline Delft Units) determined as described in British patent 1519148.

Besides obtaining a more pure protein preparation, surprisingly, the ultrafiltration behaviour of the fermentation broth improves.

Furthermore, a precipitate of inactive material is usually formed during concentration by ultrafiltration in case of the unpurified enzyme solution so removing the cell wall polymers. After ion exchange treatment, the retentate remains clear, so preventing the need for a laborious filtration step of the viscous final product. The formation of haze in detergent formulations after storage with the untreated enzyme liquid can also be avoided by this treatment. Finally, the immunogenicity of the protein preparation obtained from a fermentation broth purified by ion exchange chromatography decreases significantly.

The following test methods, analysis techniques and Examples further illustrate the invention.

## Iodine Starch Amylase Test

Thermostable bacterial amylase hydrolyses starch into dextrins and maltose. Iodine is added to the hydrolysate at increasing times of incubation and the absorbance of the colour formed is measured spectrophotometrically. The time required for the absorbance to equal a colour reference is a function of enzymatic activity.

### Reagents

- Tris-maleate buffer pH 6.6.
12.12 g tris(hydroxymethyl) aminomethane, 11.6 g maleic acid and 10.0 g calcium chloride dihydrate were dissolved in distilled water. After addition of 190 ml 0.500 N sodium hydroxide, the solution was made up to 1 liter.
- Synthetic tap water (STW) buffer solution.
To 0.95 liter of distilled water was added consecutively: 10.0 ml of a 0.198 M calcium chloride solution, 10.0 ml of 0.0689 M magnesium chloride solution, 10 ml of a 0.250 M sodium bicarbonate solution and 20 ml of the tris-maleate buffer.
- A 2% (w/v) solution of soluble starch (Merck) in 50 times diluted tris-maleate buffer.
- Iodine stock solution containing 22 g iodine and 44 g potassium iodine per liter of distilled water.
- Diluted iodine solution: 4 ml of iodine stock solution and 40 g potassium iodine dissolved in distilled water. Distilled water added up to 1 liter.
- Colour reference containing 250 g cobaltous chloride hexahydrate and 38.4 g potassium bichromate per liter in 0.01 N HCl.

### Procedure

The enzyme sample is dissolved in the STW buffer solution and diluted with the same to a concentration between 2 and 3 TAU per ml. Starting at time O exactly 10 ml of the sample solution is added to 20 ml 2% (w/v) starch solution. After 11 minutes of incubation at 30° C, 1 ml of the solution is transferred to 5 ml of the diluted iodine solution. The optical density is immediately measured at 620 nm in a 1 cm cuvet using distilled water as the blank. The procedure of transferring and measuring is repeated at 1 minute intervals until readings are found which are lower than the readings of colour reference.

The time T needed to reach the absorbance equal to that of the colour reference is established graphically.

The thermostable amylase activity in units (TAU) present in the incubation solution is calculated from $400/T$ in which:
400: mg of soluble starch in the incubation solution;
T : reaction time needed (min).

## Determination of the protein content

The protein content is determined after 5% (w/v) trichloroacetic acid precipitation according to the biuret method (H.U. Bergmeyer, Methods of enzymatic analysis, third edition, vol. II, 86-88 (1974)). Bovine serum albumin is used as a standard.

Determination of the teichoic acid content

The content of teichoic acid is determined with $^{31}$P-NMR.

Determination of the peptidoglycan components glucosamine, muramic acid and diaminopimelic acid and the teichuronic acid components galactosamine and glucuronic acid

The peptidoglycan components glucosamine, muramic acid and diaminopimelic acid and the teichuronic acid components galactosamine and glucuronic acid are determined as follows: a 1 ml sample is diluted five times with chilled water and centrifuged at 4°C for 20 minutes at 2000 *g. The supernatant is decanted and extracted twice with 5 ml petroleum ether (boiling range 60-80°C). The denatured protein, which formed a layer at the interface between the two liquids, is included in the water phase. The extracted sample is cooled to 0°C in ice and 1/10 volume 100% (w/v) trichloroacetic acid is added. After incubation at 4°C for 60 minutes the precipitate is removed by centrifugation at 4°C (20 min, 2000 *g) and the sample is filtered over a nitrocellulose filter (0.6 μm). The filtrate is extracted three times with the same volume ether to remove the trichloroacetic acid. After hydrolysis in concentrated sulfuric acid, the ether-extracted samples are measured for glucuronic acid with harmine reagent (commercially available, Sigma) essentially according to A.H. Wiarda, W.S. Allen and R. Varma, Anal. Biochem., 57, 268 (1974). For determination of peptidoglycan components and galactosamine the ether-extracted samples are hydrolysed in 6N HCl for 17 hours at 110°C in air-tight stoppered tubes. The HCl is removed by a flow of nitrogen gas at 40°C for 1.5 hour. Muramic acid, glucosamine, diaminopimelic acid and galactosamine were determined using an aminoacid analyser (kontron, liquimat III) with an elution system based on citrate buffers.

Example I

Decrease of content of cell wall polymers, increase of specific α-amylase activity based on protein, increase of specific α-amylase activity based on dry weight, improvement of ultrafiltration behaviour and reduction of the allergenicity, of α-amylase obtained from a fermentation broth by purification with ion exchange chromatography.

A fermentation broth of A containing Maxamyl®, a commercial preparation of Bacillus licheniformis available from Gist-brocades, and some impurities as described in Table 1 was purified to give the recovery sample A-1 containing Maxamyl®. The α-amylase activity, the protein content and the content of cell wall polymers teichoic acid, peptidoglycan and teichuronic acid were determined in the extracellular fluid of the fermentation broth A and in the recovery sample A-1 by the analytical methods indicated above.

Table 1 shows that in addition to protein (α-amylase) the extracellular fluid also contains the cell wall components peptidoglycan, teichoic acid and teichuronic acid. It is obvious that during the recovery these cell wall components co-fractionate with the α-amylase activity.

The recovery sample A-1 was treated, in an extra recovery step, by ion exchange chromatography as follows. A quickfit glass column (height: 33.5 cm and diameter: 5.0 cm) was used. The bed volume was 679 ml and the column was packed with IRA 958 (Amberlite, Rohm and Haas). The flow in the column was 2.14 bed volumes/h. The pH of sample A-1 was adjusted with acetic acid to 6.1 and 100 g sample was pumped into the column, followed by 1100 ml buffer (50 mM sodium acetate, pH = 6.1) and 1200 ml eluent (50 mM sodium acetate and 1 M sodium chloride, pH = 6.1). Fractions of 100 ml were collected, the product was present between the third and seventh fraction. This 500 ml product was concentrated six times by ultrafiltration in an Amicon stirred cell with a PM 10 membrane. The yield of α-amylase activity after ion exchange chromatography and ultrafiltration was more than 99%. The resulting purified fraction of A-1 was designed A-2.

The ion exchange treated and the untreated sample were analyzed for cell wall components as described above. Table 2 shows that following ion exchange chromatography, there is a drastic decrease of the cell wall polymers in the sample.

The protein content of the samples A-1 and A-2 was determined after trichloroacetic acid precipitation according to the biuret method. Bovine serum albumin was used as a standard. Table 2 shows that the ion exchange chromatography purification step resulted in a 10% increase in the specific $\alpha$-amylase activity in the protein of A-2 compared to A-1.

Duplicate samples containing 10 g of A-1 and A-2 were dried at 100°C for 16 hours to give dry weight values. Table 2 shows that following the ion exchange chromatography purification step, the specific activity based on dry weight increases. This is due to the removal of cell wall polymers and of non $\alpha$-amylase protein.

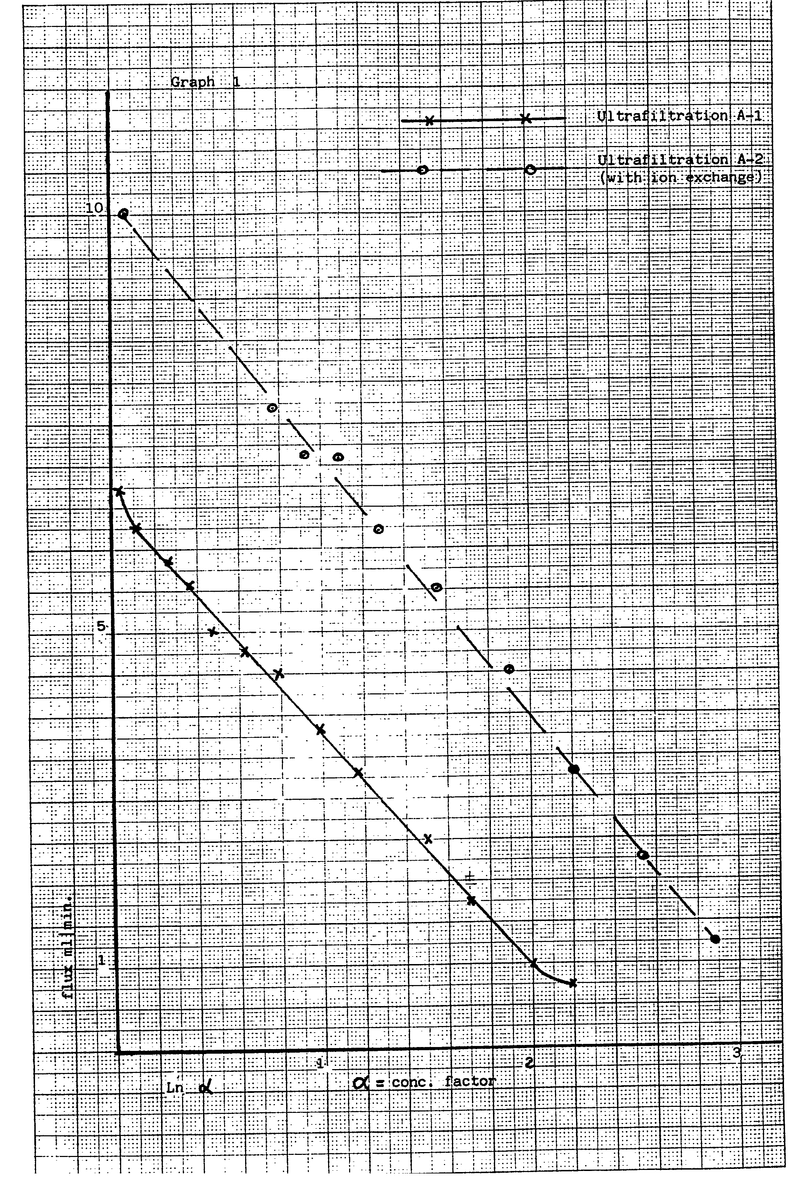

The ultrafiltration behaviour of samples A-1 and A-2 are shown in graph 1. There is an increase in the flux of ultrafiltration in A-2. Graph 1 also shows that a more concentrated $\alpha$-amylase preparation can be obtained.

Samples of A-1 and A-2 dissolved in physiological saline, were administered three times at fortnightly intervals by the intradermal route at doses of 0.1 or 1.0 $\mu$g protein per guinea-pig (eight animals per dose group).

Female guinea-pigs, Dunkin-Hartley-Pirbright origin, weighing 250-350 g were supplied by OALC 1976 Ltb-Bicester, England. Animals were individually housed and fed on Hope Farms pellets Type LC-23B and tap water ad libitum.

Assessment of response

1. Serial dilutions of serum from blood removed one week after the third intradermal injection were tested in an homologous passive cutaneous anaphylaxis (PCA) test for a quantitative determination of circulating antibody.

2. Two weeks after the third intradermal injection, guinea pigs were challenged by the intratracheal administration of enzyme (1 $\mu$g enzyme eq.) and the respiratory response scored visually.

Appropriate controls were employed at all stages of testing.

Results

The results of the PCA tests were as follows

1. Antibody titre [1]).

## Dose

| Sample | 0.1 $\mu$g | 1.0 $\mu$g |
|---|---|---|
| A-1 | 7.8 (6.7 - 8.8) | 9.6 (8.9 - 10.4) |
| A-2 | 1,0 (-2.3 - 4.2) | 9.3 (8.5 - 10.1) |

There was little difference between responses to the two test substances at the 1.0 $\mu$g dose level which, on the basis of wider experience, probably reflects a plateau reponse achieved by super-optimal dosing. On the other hand the 0.1 $\mu$g dose level of test substances proved to be more discriminating, resulting in much lower antibody titre in the serum for A-2 treated guinea-pigs. When the two test substances were compared by performing a parallel-line assay by probit analysis on the summated values of PCA-tests on the serial dilutions of serum from the 0.1 $\mu$g dose level, a highly significant potency ratio of $2^{5.4}$ ($2^{3.6}$-$2^{7.3}$) was found between A-1 and A-2. This indicates that the application of the ion-exchange

1) The values given represent the dilutions (expressed as the power of 2) and the 95% confidence intervals to induce 50% of the maximum response.
Negative values indicate that a concentration of the serum would be required to attain a 50% response.

techniques, the only difference between the method of preparation of samples A-2 and A-1, can significantly reduce the immunogenicity of these preparations.

2. Respiratory response.

The average respiratory response, scored on a scale of 0-7, was also lower in the A-2 treated group than in the A-1 treated group at the 0.1 $\mu$g dose level (0.9 vs 2.6 respectively).

## Example II

Decrease of formation of haze in a solution with detergents containing amylase by ion exchange chromatography.

A Maxamyl® broth filtrate of ± 2000 TAU/g was passed through a column with Amberlite IRA 958. Afterwards the Amberlite IRA 958 Was regenerated using a 10% NaCl solution. The teichoic acid free Maxamyl® solution was concentrated to 15000 TAU/g final product and thereafter stabilised with propylene glycol to 50% (w/v).

Treated and untreated final enzyme solution was added to a haze sensitive detergent mixture (containing a non-ionic polyethylene oxide alcohol (16% (w/v)), sodium dodecylbenzene sulphonate (3.5% (w/v)), ethanol (5% (w/v)) and sodium formate (3% (w/v)) in water) and stored for 50 hours at 43° C. The IRA 958-untreated samples showed a heavy precipitate formation, while no precipitate was found in the samples treated with the ion exchange.

## Example III

Decrease of content of cell wall polymers of protease obtained from a fermentation broth by purification with ion exchange chromatography.

In a fermentation broth of Maxacal® obtained as described in British patent 1519418 (P-1) the alkaline protease activity and the protein content were determined by the analytical methods indicated above. The presence of pyruvate ketales containing cell wall polymers was determined with $^1$H-NMR, after ultrafiltration with a YM10 membrane.

Sample P-1 was treated, in an extra recovery step, by ion exchange chromatography essentially as described in Example I. In the resulting purified fraction of P-1 (P-2) the pyruvate ketales containing cell wall polymers could not be detected at all.

The protein content of the samples P-1 and P-2 were determined as described in example I. Table 3 shows that the ion exchange purification step also resulted in a 5% increase in the specific protease activity based in the protein of P-2 compared to P-1.

## Table 2

| Sample | ion exchange | amylase $[10^6 TAU/1]$ | TA [mM] | GlcNH$_2$ [mM] | MurNH$_2$ [mM] | DAP [mM] | GalNH$_2$ [mM] |
|--------|-------------|----------|---------|---------|---------|---------|---------|
| A-1 | − | 17.7 | 148 | 12.1 | 6.6 | 10.2 | 50.6 |
| A-2 | + | 20.5 | 3.4 | 1.2 | <0.3 | 1.9 | 1.0 |

| Sample | GlcA [mM] | protein [g/l] | spec. act. $[10^3 TAU/g$ protein] | drw [g/l] | spec. act. $[10^3 TAU/g$ drw] |
|--------|-----------|---------------|-------------|-----------|-------------|
| A-1 | 30.6 | 53 | 334 | 191 | 93 |
| A-2 | <0.1 | 55 | 373 | 72 | 285 |

EP 0 322 082 A1

EP 0 322 082 A1

Table 1

| Sample | Protein [g/$10^6$ TAU] | TA [mMol/$10^6$ TAU] | GlcNH$_2$ [mMol/$10^6$ TAU] | MurNH$_2$ [mMol/$10^6$ TAU] | DAP [mMol/$10^6$ TAU] | GalNH$_2$ [mMol/$10^6$ TAU] | GlcA [mMol/$10^6$ TAU] |
|---|---|---|---|---|---|---|---|
| A | 3.8 | 8.6 | 1.4 | 0.25 | 0.56 | 1.1 | 2.3 |
| A-1 | 3.0 | 8.4 | 0.7 | 0.37 | 0.38 | 2.9 | 1.7 |

Abbreviations used in Tables 1, 2 and 3.

TA : teichoic acid

spec. act : specific activity

TAU : thermostable amylase activity unit as defined

ADU : alkaline Delft unit

GlcNH$_2$ : glucosamine

MurNH$_2$ : muramic acid

DAP : diaminopimelic acid

GalNH$_2$ : galactosamine

GlcA : glucuronic acid

drw : dry weight

Table 3

| Sample | spec. act. [$10^6$ ADU/g protein] |
|---|---|
| P-1 | 8.9 |
| P-2 | 9.4 |

## Claims

1. Industrial extracellular protein from the fermentation of a <u>Bacillus</u> strain substantially free from cell wall polymers.

2. Industrial extracellular protein according to claim 1, which is an enzyme.

3. Industrial enzyme according to claim 2, which is α-amylase.

4. Industrial α-amylase according to claim 3, which is a thermostable amylase.

5. Industrial α-amylase according to claim 3 or 4 characterized in that it contains less than 0.2 mMol teichoic acid per $10^6$ TAU, less than 0.1 mMol glucosamine per $10^6$ TAU, less than 0.03 mMol muramic acid per $10^6$ TAU, less than 0.2 mMol diaminopimelic acid per $10^6$ TAU, less than 0.1 mMol galactosamine per $10^6$ TAU and less than 0.1 mMol glucuronic acid per $10^6$ TAU.

6. Industrial enzyme according to claim 2, which is protease.

7. Industrial protease according to claim 6, which is active in alkaline medium.

8. A process for the preparation of an industrial protein as defined in any one of the preceding claims characterized in that cell wall polymers are removed from the extracellular protein by ion exchange.

9. A process for the preparation of a purified industrial protein according to claim 8, which protein is an enzyme.

Graph 1

Ultrafiltration A-1

Ultrafiltration A-2
(with ion exchange)

flux ml/min.

Ln α          α = conc. factor

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | STARCH/STÄRKE, vol. 31, no. 3, November 1979, pages 86-92, Verlag Chemie, GmbH, Weinheim, DE; J.P. CHIANG et al.: "Purification and characterization of a thermostable alpha-amylase from Bacillus licheniformis" * Page 86, paragraph 1 - page 87, paragraph 2.3; page 89; summary * | 1-5,8,9 | C 12 P 21/02<br>C 12 N 9/00<br>C 12 N 9/28<br>C 12 N 9/54 |
| X | MICROBIOLOGY ABSTRACTS, vol. 14, no. 11, November 1979, pages 9-10, abstract no. 8300-A14, Philadelphia, PA, US; J.P. CHIANG et al.: "Purification and characterization of a thermostable alpha-amylase from Bacillus licheniformis", & STARKE, 31(3), 86-92 (1979) | 1-5 | |
| X | EP-A-0 193 046 (MILES LABORATORIES, INC.) * Page 5, line 8 - page 6, line 1; page 9, line 18 - page 11, line 15; examples 1,2; claims 1,10 * | 1-7 | |
| | | | TECHNICAL FIELDS SEARCHED (Int. Cl.4) |
| X | EP-A-0 214 531 (MILES LABORATORIES, INC.) * Page 5, line 10 - page 6, line 2; page 8, lines 8-12; examples 1-4; claims 9-11 * | 1-7 | C 12 N |
| X | GB-A- 810 566 (NAASUE & CO. LTD) * Page 1, line 15 - page 2, line 64; examples 1,2; claims 1-4 * | 1,2,6-9 | |
| X | EP-A-0 220 921 (GENEX CORP.) * Page 2, lines 28-36; page 2, lines 53-56; example 2 *<br>---      -/- | 1,2,6-9 | |

The present search report has been drawn up for all claims

| Place of search | Date of completion of the search | Examiner |
|---|---|---|
| THE HAGUE | 14-03-1989 | GROENENDIJK M.S.M. |

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 232 169  (GENEX CORP.)<br>* Page 3, lines 14-24; page 4, lines 26-33; example 2 *<br>--- | 1,2,6-9 | |
| A | EP-A-0 244 998  (ROHM & HAAS CO.)<br>* Page 2, line 5 - page 4, line 1; table 1, examples 6-15; claims 1-5,7-11 *<br>----- | 8,9 | |

TECHNICAL FIELDS
SEARCHED (Int. Cl.4)

The present search report has been drawn up for all claims

| Place of search | Date of completion of the search | Examiner |
|---|---|---|
| THE HAGUE | 14-03-1989 | GROENENDIJK M.S.M. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...............................................................................
& : member of the same patent family, corresponding
   document

EPO FORM 1503 03.82 (P0401)